Europäisches Patentamt

(19) European Patent Office

Office européen des brevets

(11) Veröffentlichungsnummer: **0 110 369**
**B1**

(12) **EUROPÄISCHE PATENTSCHRIFT**

(45) Veröffentlichungstag der Patentschrift:
30.04.86

(21) Anmeldenummer: 83111908.6

(22) Anmeldetag: 28.11.83

(51) Int. Cl.⁴: **C 07 C 50/24,** C 07 C 46/10

(54) Verfahren zur Reinigung von rohem Monochlor- und Monobromanthrachinon.

(30) Priorität: 29.11.82 CH 6922/82

(43) Veröffentlichungstag der Anmeldung:
13.06.84 Patentblatt 84/24

(45) Bekanntmachung des Hinweises auf die Patenterteilung:
30.04.86 Patentblatt 86/18

(84) Benannte Vertragsstaaten:
CH DE FR GB LI

(56) Entgegenhaltungen:
CH - A - 157 656
DE - A - 2 531 929
DE - B - 2 458 022

PATENT ABSTRACTS OF JAPAN, unexamined applications, Field C, Vol. 5, Nr. 85, 3. Juni 1981 THE PATENT OFFICE JAPANESE GOVERNMENT, Seite 153 C 57

(73) Patentinhaber: **CIBA-GEIGY AG, Klybeckstrasse 141, CH-4002 Basel (CH)**

(72) Erfinder: **Somlo, Tibor, Dr., Florastrasse 8, CH-4127 Birsfelden (CH)**
Erfinder: **Regli, Johann, Passwangstrasse 4, CH-4127 Birsfelden (CH)**

(74) Vertreter: **Zumstein, Fritz jun., Dr. et al, Dr. F. Zumstein sen. Dr. E. Assmann Dr. R. Koenigsberger Dipl.-Ing. F. Klingseisen Dr. F. Zumstein jun. Bräuhausstrasse 4, D-8000 München 2 (DE)**

## Beschreibung

Die Erfindung betrifft ein Verfahren zur Reinigung von rohem Monochlorund Monobromanthrachinon.

Halogenanthrachinone, insbesondere 1-Chloranthrachinon, sind wichtige Ausgangssubstanzen für die Farbatoffsynthese. Dabei ist die Reinheit dieser Ausgangsmaterialien von entscheidender Bedeutung für die Reproduzierbarkeit des Farbtons und für das Echtheitsniveau der daraus hergestellten Farbstoffe.

Bekanntlich enthalten rohe Chlor- und Bromanthrachinone stets eine Reihe von unerwünschten, schwer abzutrennenden Nebenprodukten. Derartige rohe Reaktionsgemische können z.B. durch fraktionierte Vakuumdestillation gereinigt werden (DE-OS 24 58 022 und DE-OS 25 31 929). Dieses Verfahren weist jedoch wesentliche Nachteile auf. So erfordert die Destillation Vakuumbedingungen und relativ hohe Temperaturen (Kopftemperatur 230 bis 270°C; Sumpftemperatur um 300°C) bei denen sich das zu reinigende Halogenanthrachinon bereits teilweise zersetzt. Ferner ist wegen der starken Kristallisations- und Sublimationsneigung etwa des 1-Chloranthrachinons, eine Beheizung sämtlicher mit dem produkt in Berührung kommender Apparateteile erforderlich, was aufwendige apparative Massnahmen bedingt. Auch müssen die Halogenanthrachinone vor der Destillation einer Vorreinigung unterworfen, z.B. säurefrei gewaschen werden und nach der Destillation ist ein weiterer Arbeitsgang erforderlich, um das als Schmelze anfallende Produkt in eine leicht zu handhabende Form, wie z.B. ein Granulat, zu überführen.

Die Aufgabe bestand demnach darin, ein Reinigungsverfahren für rohe Halogenanthrachinone zu entwickeln, das die genannten Nachteile nicht aufweist.

Gefunden wurde, dass man Monochlor- und Monobromanthrachinon in hoher, technisch brauchbarer Reinheit (95- bis 99%ig) auf einfache Art und Weise erhält, indem man rohes Monochlor- bzw. Monobromanthrachinon (80- bis 90%ig) in einem solchen Verhältnis mit einem apolaren aliphatischen Lösungsmittel mischt, dass in der Hitze mehr als die Hälfte in Lösung geht und der die Verunreinigungen enthaltende Rest eine eutektische Schmelze bildet.

Von diesem flüssigen 2-Phasensystem lässt sich die eutektische Schmelze als untere phase leicht abtrennen, während das Monochlor- bzw. Monobromanthrachinon durch Abkühlen der die obere phase bildenden gesättigten Lösung in reiner, kristalliner, gut zu handhabender Form isoliert wird.

Rohes Monochlor- bzw. Monobromanthrachinon mit einem Gehalt von ca. 80 bis 90% lässt sich nach dem Verfahren gemäss vorliegender Erfindung weitgehend von Nebenprodukten, wie halogenierten Bisanthrachinonylen, befreien, da diese zusammen mit bestimmten Anteilen an Monohalogenanthrachinon überraschenderweise eine leicht abtrennbare eutektische Schmelze bilden. Naturgemäss richtet sich der angestrebte Reinheitsgrad nach den Anforderungen, die an die Halogenanthrachinone bei deren Weiterverarbeitung zu Farbstoffen gestellt werden. Je nach Qualitätsanforderung muss mehr oder weniger Halogenanthrachinon mit der eutektischen Schmelze abgetrennt werden.

Bevorzugt wird das Verfahren zur Reinigung von 1-Brom- und vor allem 1-Chlor-anthrachinon eingesetzt. Anwendbar ist es jedoch mit gleich gutem Reinigungseffekt auch auf Anthrachinone, die in 2-Stellung durch Chlor bzw. Brom substituiert sind.

Als apolare aliphatische Lösungsmittel kommen z.B. gegebenenfalls halogenierte Kohlenwasserstoffe mit einem Siedepunkt oberhalb von 125°C in Frage. Als besonders geeignet haben sich Gemische aus unverzweigten und/oder verzweigten aliphatischen Kohlenwasserstoffen erwiesen, deren Siedebereich in einem Temperaturintervall von 130 bis 220°C liegt, und die frei sind von Aromaten und Naphthenen. Besonders geeignet sind Isoparaffingemische mit Siedebereichen von 155 bis 175°C, 170 bis 190°C bzw. 190 bis 210°C, wie sie z.B. von ESSO unter den Handelsnamen Isopar G, H bzw. L vertrieben werden.

Bei der praktischen Durchführung des erfindungsgemässen Reinigungsverfahrens geht man zweckmässiger Weise so vor, dass man das rohe Monochloroder Monobromanthrachinon in fester Form oder bevorzugt als Schmelze in das apolare Lösungsmittel einträgt. Anschliessend wird das Rohprodukt in der Hitze gelöst, wobei das Lösungsmittel bereits zu Anfang eine Temperatur von über 130°C jedoch unterhalb der Schmelztemperatur des reinen Monochlor- bzw. Monobromanthrachinons, vorteilhaft zwischen 140 und 180°C, aufweist oder erst nach Zugabe der zu reinigenden Verbindung auf eine Temperatur im genannten Bereich erhitzt wird. Wichtig dabei ist, dass Rohprodukt und Lösungsmittel in einem solchen Verhältnis zusammengegeben werden, dass sich auch in der Hitze nicht das gesamte Monochlor- bzw. Monobromanthrachinon löst, sondern ein gewisser Teil mit den Verunreinigungen als eutektische Schmelze eine zweite Phase bildet.

Beispielsweise trägt man 90%ige Ware in soviel Lösungsmittel ein, dass sich etwa 80% davon in der Hitze lösen und 10% des nicht gelösten Chlor- bzw. Bromanthrachinons zusammen mit den unlöslichen Nebenprodukten eine eutektische Schmelze bildet.

Die Temperatur des Lösungsmittels muss somit ausreichend hoch sein, dass der nicht gelöste Rest im geschmolzenen Zustand vorliegt und sich von der gesättigten Lösung des Chlor- bzw. Bromanthrachinons als zweite phase abscheidet. Nach Abtrennen dieser phase lässt man die gesättigte Lösung auf eine Temperatur von vorteilhaft 20 bis 60°C unter Rühren abkühlen, wobei das Chlor- bzw. Bromanthrachinon in reiner Form ausfällt.

Zweckmässigerweise werden Temperatur und Mengenverhältnisse so gewählt, dass die eutektische Schmelze etwa zu zweidrittel aus Halogenanthrachinon und einem Drittel aus Verunreinigungen besteht. Natürlich kann dieses Mengenverhältnis in einem gewissen Bereich schwanken, wobei als untere bzw. obere Grenze ein Gehalt von 30 bzw. 90 Gewichtsprozent, vorzugsweise 50 bis 75 Gewichtsprozent, Halogenanthrachinon anzusehen ist. Das nach mehreren Reinigungsoperationen angesammelte Eutektikum kann aufgearbeitet werden; durch Kristallisation oder Destillation.

Mit Hilfe des erfindungsgemässen Verfahrens wird rohes Monochlor- und Monobromanthrachinon, unabhängig von seiner provenienz nahezu vollständig von Nebenprodukten befreit. Gereinigt werden kann beispielsweise 1-Chlor-anthrachinon, das nach A. Fischer ausgehend von Anthrachinonsulfonsäure durch Austausch der Sulfonsäuregruppe erhalten wurde (siehe Ullmann, 4. Auflage, Band 7, Seite 589) oder auch durch Umsetzen von Nitroanthrachinon mit elementarem Chlor hergestellt wurde (DRp 252.578 und 254.450).

Da je nach Grad der Verunreinigung, mit den Nebenprodukten etwa die doppelte Menge an Chlor- bzw. Bromanthrachinon mitabgetrennt wird, wird man naturgemäss einem Herstellungsverfahren für Halogenanthrachinone den Vorzug geben, das bereits eine relativ hochprozentige Ware liefert.

Leicht zu reinigendes rohes Monochloranthrachinon mit einem niedrigen Gehalt an Nebenprodukten wird nach einem bevorzugten Verfahren so hergestellt, dass man Nitroanthrachinon bei einer Temperatur von 180 bis 300°C, vorzugsweise 200 bis 270°C, mit elementarem Chlor umsetzt, wobei man in Gegenwart eines, unter den Reaktionsbedingungen inerten Lösungsmittels mit einem Siedepunkt über 180°C arbeitet. Das Lösungsmittel wird in einer Menge von 1 bis 20 Gewichtsprozent, vorzugsweise 5 bis 10 Gewichtsprozent, bezogen auf Nitroanthrachinon, zugesetzt.

Die Reaktion kann z.B. in einem Rührkessel durchgeführt werden, der mit einem Begasungsrührer ausgerüstet ist. Als Lösungsmittel kommen vor allem Nitrobenzol, Sulfolan oder Hexachlorbutadien in Frage. Das Lösungsmittel verdampft während der Reaktion und zurück bleibt die Chloranthrachinon-Schmelze, die ohne Zwischenisolierung und Vorreinigung zur Reinigung direkt in das apolare Lösungsmittel eingetragen werden kann.

Die Erfindung wird durch die folgenden Beispiele erläutert.

**Beispiel 1**

In einem 2 1/2 1 Sulfierkolben mit Untenauslauf werden 1500 g eines Isoparaffingemisches mit Siedegrenzen von 155 bis 173°C (Isopar G / ESSO) vorgelegt, darin 380 g rohes, 88%iges 1-Chloranthrachinon eingetragen, das Gemisch aufgeheizt und eine Stunde bei 140 bis 145°C gerührt. Danach wird der Rührer abgestellt, ein schwarzbraunes Oel, das sich als untere Phase abgetrennt hat, abgelassen und die hellgelbe Lösung abgekühlt. Dabei kristallisiert das reine 1-Chloranthrachinon in hellgelben Kristallen aus. Bei 50°C werden diese abgenutscht und im Vakuum bei 110°C getrocknet. Auf diese Weise werden 300 bis 310 g 1-Chloranthrachinon mit einem Gehalt von 96 $\pm$ 1% in einer direkt gebrauchsfertigen Form erhalten.

**Beispiel 2**

In einem 1 1/2 1 Reaktionsgefäss werden 200 Gewichtsteile Nitrobenzol vorgelegt und darin bei 200 bis 210°C 1771 Gewichtsteile 1-Nitroanthrachinon eingetragen. Darauf werden bei 215 bis 220°C innerhalb von 3 bis 4 Stunden über einen Begasungsrührer ca. 520 Gewichtsteile Chlorgas eingeleitet, wobei das Nitrobenzol über einen Abscheider abdestilliert. Ist die Umsetzung beendet, wird die Schmelze durch kurzes Ausblasen mit Stickstoff von Gas- und Nitrobenzol-Spuren befreit, auf 170°C gekühlt. Anschliessend lässt man die Schmelze in 6800 Gewichtsteile eines Isoparaffingemisches mit Siedegrenzen von 190 bis 211°C (Isopar L, ESSO) einlaufen, das zuvor auf eine Temperatur von 135 bis 140°C erwärmt wurde. Das Gemisch wird eine Stunde bei 140 bis 145°C gerührt, dann wird der Rührer abgestellt und die untere braunschwarze Oelschicht durch den Untenauslauf abgetrennt. Nach einer halben Stunde Rühren bei 135°C wird die Abtrennung wiederholt. Die hellgelbe Lösung wird darauf bis 50°C abgekühlt, die dabei ausfallenden Kristalle werden abgenutscht und bei 120°C im Vakuum bis zur Gewichtskonstanz getrocknet. Erhalten werden 1360 g 1-Chloranthrachinon mit einem Gehalt von 96 $\pm$ 1%.

**Beispiel 3**

In einem 2 1/2 1 Sulfierkolben mit Untenauslauf werden 1500 Gewichtsteile eines Isoparaffingemisches mit Siedegrenzen von 182 bis 212°C (Shellsol T, Shell Chemie) vorgelegt, darin 400 Gewichtsteile rohes 92%iges Bromanthrachinon eingetragen, das Gemisch aufgeheizt und eine Stunde bei 175°C gerührt. Danach wird der Rührer abgestellt, ein schwarzbraunes Oel, das sich als untere Phase abgetrennt hat, abgelassen und die hellgelbe Lösung abgekühlt. Dabei kristallisiert das reine 1-Bromanthrachinon in grellgelben Kristallen aus. Bei 50°C werden diese abgenutscht und im Vakuum bei 110°C getrocknet. Auf diese Weise werden 330 g 1-Bromanthrachinon mit einem Gehalt von 98 bis 99% erhalten.

**Patentansprüche**

1. Verfahren zur Reinigung von rohem Monochlor- und Monobromanthrachinon, dadurch gekennzeichnet, dass man das durch Nebenprodukte verunreinigte Monochlor- bzw.

Monobromanthrachinon in einem solchen Verhältnis mit einem apolaren aliphatischen Lösungsmittel mischt, dass in der Hitze mehr als die Hälfte in Lösung geht und der die Verunreinigungen enthaltende Rest als eutektische Schmelze eine zweite untere phase bildet, die eutektische Schmelze abtrennt, sodann die gesättigte Lösung abkühlen lässt und das sich in reiner Form abscheidende Monochlor- bzw. Monobromanthrachinon isoliert.

2. Verfahren gemäss Anspruch 1, dadurch gekennzeichnet, dass man rohes 1-Brom-, insbesondere 1-Chloranthrachinon verwendet.

3. Verfahren gemäss Anspruch 1, dadurch gekennzeichnet, dass man als apolares aliphatisches Lösungsmittel gegebenenfalls halogenierte Kohlenwasserstoffe mit einem Siedepunkt oberhalb von 125°C verwendet.

4. Verfahren gemäss Anspruch 1, dadurch gekennzeichnet, dass man ein Gemisch unverzweigter und/oder verzweigter aliphatischer Kohlenwasserstoffe verwendet, deren Siedebereich in einem Temperaturintervall von 130 bis 220°C liegt.

5. Verfahren gemäss Anspruch 1, dadurch gekennzeichnet, dass das Lösungsmittel bei Abscheidung der eutektischen Schmelze eine Temperatur aufweist, die oberhalb 130°C und unterhalb der Schmelztemperatur des reinen Monochlor- bzw. Monobromanthrachinons liegt.

6. Verfahren gemäss Anspruch 1, dadurch gekennzeichnet, dass die eutektische Schmelze zu 30 bis 90 Gewichtsprozent, vorzugsweise 50 bis 75 Gewichtsprozent, aus Monochlor- bzw. Monobromanthrachinon besteht.

7. Verfahren gemäss Anspruch 1, dadurch gekennzeichnet, dass man die Lösung des Monochlor- bzw. Monobromanthrachinons nach Abtrennen der eutektischen Schmelze auf eine Temperatur von 20 bis 60°C abkühlen lässt.

## Claims:

1. A process for purifying crude monochloro- or monobromoanthraquinone, which comprises mixing monochloro- or monobromoanthraquinone which contains by-products as impurities with a non-polar aliphstic solvent in such a rstio that at elevated temperature more than half of the crude product goes into solution and the remainder which contains the impurities forms a eutectic melt as second lower phase, separating said melt, allowing the saturated solution to cool, and isolating the pure precipitated monochloro- or monobromoanthraquinone.

2. A process according to claim 1, for purifying crude 1-bromo-anthraquinone, in particular 1-chloroanthraquinone.

3. A process according to claim 1, wherein hydrocarbon or a halogenated hydrocarbon with a boiling point above 125° C is used as non-polar aliphatic solvent

4. A process according to claim 1, which comprises the use of a mixture of unbranched and/or branched aliphatic hydrocarbons having a boiling range from 130° to 220° C.

5. A process according to claim 1, wherein the solvent, upon separation of the eutectic melt, has a temperature which is above 130° C and below the melt temperature of the pure monochloro- or monobromoanthraquinone.

6. A process according to claim 1, wherein the eutectic melt comprises up to 30 to 90 % by weight, preferably from 50 to 75 % by weight, of monochloro- or monobromoanthraquinone.

7. A process according to claim 1, wherein the solution of the monochloro- or monobromoanthraquinone is cooled to a temperature in the range from 20° to 60° C after separation of the eutectic melt.

## Revendications

1. Procédé pour la purification de monochloro- et monobromoanthraquinone brute, caractérisé en ce qu'on mélange la monochloro- ou monobromoanthraquinone, contaminée par des sous-produits, à un solvant aliphatique apolaire selon une proportion telle que, à chaud , plus de la moitié passe en solution, et le reste, contenant les impuretés, forme en tant que masse fondue eutectique une deuxième phase inférieure, on sépare la masse fondue eutectique, puis on laisse refroidir la solution saturée et on isole la monochloro ou monobromoanthraquinone qui se sépare sous forme pure.

2. Procédé selon la revendication 1, caractérisé en ce qu'on utilise de la 1-bromo-, en particulier de la 1-chloro-anthraquinone brute.

3. Procédé selon la revendication 1, caractérisé en ce qu'on utilise en tant que solvant aliphatique apolaire des hydrocarbures éventuellement halogénés ayant un point d'ébullition supérieur à 125°C.

4. Procédé selon la revendication 1, caractérisé en ce qu'on utilise un mélange d'hydrocarbures aliphatiques à chaîne droite et/ou ramifiée, dont le domaine d'ébullition se trouve dans l'intervalle de températures de 130 à 220°C.

5. Procédé selon la revendication 1, caractérisé en ce que le solvant, lors de la séparation de la masse fondue eutectique, présente une température qui est supérieure à 130°C et est inférieure à la température de fusion de la monochloro- ou monobromoanthraquinone pure.

6. Procédé selon la revendication 1, caractérisé en ce que la masse fondue eutectique est constituée pour 30 à 90 % en poids, de préférence pour 50 à 75 % en poids, de monochloro- ou monobromoanthraquinone.

7. Procédé selon la revendication 1, caractérisé en ce que, après séparation de la masse fondue eutectique, on laisse refroidir la solution de monochloro- ou monobromoanthraquinone à une température de 20 à 60°C.